# EUROPEAN PATENT APPLICATION

(11) **EP 2 902 485 A1**
(43) Date of publication of application: **05.08.2015**
(21) Application number: 13841603.7
(22) Date of filing: 27.09.2013
(51) Int. Cl.: C12N 7/02, A61K 39/29, A61P 31/14, C12N 7/06, C12Q 1/02, C12N 15/09

(54) **HEPATITIS C VIRUS PARTICLES FORMATION PROMOTER, AND METHOD FOR PRODUCING HEPATITIS C VIRUS PARTICLES**

(30) Priority: 28.09.2012 JP 2012215474
(71) Applicant: National University Corporation Kobe University, Kobe-shi, Hyogo 657-8501 (JP); Toray Industries, Inc., Tokyo 103-8666 (JP); Tokyo Metropolitan Institute of Medical Science, Tokyo 156-0057 (JP); University of Indonesia, Depok 16424 (ID); Indonesian Institute Of Sciences (LIPI), Jakarta 12710 (ID)
(72) Inventor: HOTTA, Hak, Kobe-shi Hyogo 657-8501 (JP); AOKI, Chie, Kobe-shi Hyogo 657-8501 (JP); WAKITA, Takaji, Tokyo 169-0073 (JP); PRATIWI, Soedarmono, Jakarta 10430 (ID); RATNA, Sitompul, Jakarta 10430 (ID); LUKMAN, Hakim, Jakarta 12710 (ID); LEONARDUS, Kardono, Serpong 15314 (ID)
(74) Representative: Kador & Partner
(86) International application number: PCT/JP2013/076414
(87) International publication number: WO 2014/051111

(57) **Abstract**

Provided are a HCV particle formation promoter capable of promoting formation of HCV particles in culture cells, and a method of enhancing production of HCV particles. Also provided are a method of evaluating an anti-HCV agent candidate substance, and a method of producing a HCV vaccine. The HCV particle formation promoter comprises as an active ingredient a statin or a pharmaceutically acceptable salt thereof. Addition of the statin or pharmaceutically acceptable salt thereof to HCV-infected culture cells can promote formation of infectious HCV particles and enhance production of the particles. In addition, an anti-HCV agent candidate substance is evaluated by culturing HCV-infected cells in the presence of the HCV particle formation promoter and the anti-HCV agent candidate substance. Further, a HCV vaccine is produced by using HCV particles produced by the method of enhancing production of HCV particles.

## Description

### TECHNICAL FIELD

The present invention relates to a hepatitis C virus (HCV) particle formation promoter for promoting formation of HCV particles in HCV-infected culture cells, and to a method of enhancing production of HCV particles.

The present application claims priority from Japanese Patent Application No. 2012-215474, which is incorporated herein by reference.

### BACKGROUND ART

Since a HCV gene was cloned in 1989, establishment of a method of producing a virus growth cell system, involving infecting culture cells with HCV from hepatitis C patient serum, has been the biggest theme in HCV research.

There are as many as 200 million HCV carriers worldwide, including about 2 million HCV carriers in Japan. HCV-infected patients are at a very high risk of developing chronic hepatitis. In some of the patients, chronic hepatitis progresses to liver cirrhosis and liver cancer. That is, HCV is a causative virus that causes severe diseases. At present, a treatment method involving uses of interferon (IFN) and ribavirin, which is an antiviral drug, in combination has been often taken for many hepatitis C patients. However, there are many HCV-infected patients for whom IFN is less effective. Thus, there is a problem in that the treatment method has low therapeutic effects on such patients. In addition, it has been revealed that IFN exhibits strong side effects on patients. Therefore, there is an urgent need for development of a novel therapeutic drug and novel vaccine directed to prevention of HCV infection spread and eradication of HCV.

Meanwhile, at an animal experiment level, an infectious clone of HCV could grow only in chimeric mice transplanted with chimpanzee or human liver cells. However, the clone made little contribution to research because acquirement of a virus from a patient specimen that could easily infect culture cells was not achieved, and utilization of chimpanzees did not become popular in terms of an ethical problem and cost. It was reported that a HCV subgenomic RNA replicon system capable of replicating and growing a

HCV subgenome in culture cells was produced (Non Patent Literatures 1 to 4 and Patent Literatures 1 and 2). This enabled a replication mechanism of HCV to be analyzed using culture cells. Such HCV subgenomic RNA replicon is obtained by replacing a structural protein-encoding region present downstream of HCV IRES in a 5' untranslated region of HCV genomic RNA by a neomycin-resistant gene and EMCV-IRES linked downstream thereof. It was demonstrated that when the RNA replicon was introduced into human liver cancer cells Huh7 and the cells were cultured in the presence of neomycin, the RNA replicon replicated autonomously in the Huh7 cells.

After that, it was reported that a HCV full-genomic RNA replicon in which HCV full-genomic RNA replicated autonomously was produced, and that an infection growth system reflecting a life cycle of HCV (from virus adsorption and entry to formation of virus particles and release of the particles out of cells) was produced (Non patent Literatures 5 to 7). In order to more effectively develop a vaccine or the like, there is a demand for a system in which HCV particles are formed in infected cells and released out of the cells more effectively.

### Citation List

### Non Patent Literature

Non Patent Literature 1: Science, 285:110-113, 1999
Non Patent Literature 2: Science, 290:1972-74, 2000
Non Patent Literature 3: J. Virol., 75:12047-57, 2001
Non Patent Literature 4: Gastroenterology, 125:1808-17, 2003
Non Patent Literature 5: Nat. Med., 11:791-796, 2005
Non Patent Literature 6: Science, 309:623-626, 2005
Non Patent Literature 7: Proc. Natl. Acad. Sci. USA, 102:9294-9299, 2005

### Patent Literature

Patent Literature 1: JP 2001-17187 A
Patent Literature 2: WO 2004/104198 A1

### SUMMARY OF INVENTION

### Problem to be Solved by Invention

An object of the present invention is to provide a HCV particle formation promoter capable of promoting formation of a HCV particle in a culture cell, and a method of enhancing production of HCV particles. Another object of the present invention is to provide a method of evaluating an anti-HCV agent candidate substance, and a method of producing a HCV vaccine.

### Means for Solving Problem

The inventors of the present invention have made extensive studies in order to achieve the objects. As a result, the inventors have found that a statin or a pharmaceutically acceptable salt thereof effectively promotes formation of HCV particles. Thus, a HCV particle formation promoter according to one embodiment of the present invention has been completed. In addition, the inventors have found that an anti-HCV agent candidate substance can be evaluated by culturing a HCV-infected cell in the presence of the HCV particle formation promoter and the anti-HCV agent candidate substance. Thus, a method of evaluating an anti-HCV agent candidate substance according to one embodiment of the present invention has been completed. Further, the inventors have found that a HCV vaccine can be produced using a HCV particle produced by a method of enhancing production of HCV particles. Thus, a method of producing a HCV vaccine according to one embodiment of the present invention has been completed.

That is, the present invention includes the following.
1. A HCV particle formation promoter, comprising as an active ingredient a statin or a pharmaceutically acceptable salt thereof.
2. The HCV particle formation promoter according to the above-mentioned item 1, wherein the statin comprises one or more statins selected from the group consisting of lovastatin, fluvastatin, simvastatin, atorvastatin, and pravastatin.
3. The HCV particle formation promoter according to the above-mentioned item 1 or 2, wherein the HCV particle formation promoter comprises a culture extract of a microorganism capable of producing a statin or a pharmaceutically acceptable salt thereof.
4. The HCV particle formation promoter according to the above-mentioned item 3, wherein the microorganism is a filamentous fungus belonging to a genus *Aspergillus.*
5. A method of enhancing production of HCV particles, wherein the method comprises culturing a HCV-infected cell in a presence of the HCV particle formation promoter according to any one of the above-mentioned items 1 to 4.
6. The method of enhancing production of HCV particles according to the above-mentioned item 5, wherein the culturing is performed with addition of the HCV particle formation promoter according to any one of the above-mentioned items 1 to 4 after formation of a HCV protein in the HCV-infected cell.
7. A method of producing a HCV vaccine, wherein the method comprises inactivating a HCV produced by the method of enhancing production of HCV particles according to the above-mentioned item 5 or 6.
8. A HCV vaccine, which is produced by the method of producing a HCV vaccine according to the above-mentioned item 7.
9. A method of evaluating an anti-HCV agent candidate substance,
   wherein the method comprises:
   culturing a HCV-infected cell together with an anti-HCV agent candidate substance in a presence of the HCV particle formation promoter according to any one of the above-mentioned items 1 to 4; and
   evaluating the anti-HCV agent candidate substance for an ability to inhibit formation of HCV particles.
10. The method of evaluating an anti-HCV agent candidate substance according to the above-mentioned item 9, wherein the method comprises the steps of:
   1) adding the HCV particle formation promoter according to any one of the above-mentioned items 1 to 4 and an anti-HCV agent candidate substance to a HCV-infected cell;
   2) culturing the HCV-infected cell; and
   3) evaluating the anti-HCV agent candidate substance for an ability to inhibit formation of a HCV particle by measuring an amount of the HCV particles after the culturing.

### ADVANTAGEOUS EFFECT OF INVENTION

The formation of the HCV particle can be promoted, and the production efficiency of the HCV particles can be improved, by culturing the HCV-infected cell using the HCV particle formation promoter of the present invention. According to the methods of the present invention, the production of the HCV particles in the HCV-infected culture cell can be increased, and the anti-HCV agent candidate substance can be evaluated. Further, the HCV vaccine can be efficiently produced using the HCV particles to be obtained by the present invention.

### BRIEF DESCRIPTION OF DRAWINGS

FIG. 1 illustrates a flowchart of purification from an *Aspergillus terreus* cell extract as a production example of a HCV particle formation promoter of the present invention. In the figure, a growth inhibitory concentration (IC₅₀) and cytotoxicity (CC₅₀) for each solution in each purification step are shown (Example 1).
FIG. 2 is a graph showing measurement results of a virus titer in the case of adding the HCV particle formation promoter of the present invention to culture cells (Example 2).
FIG. 3 is a graph showing results of a study on the timing of addition of the HCV particle formation promoter to culture cells after HCV infection (Example 3).
FIG. 4 is a graph showing results of a study on the timing of addition of the HCV particle formation promoter to culture cells after HCV infection (Example 4).
FIG. 5 shows measurement results of virus particles formed inside and outside culture cells in the case of adding the HCV particle formation promoter of the present invention to the cells (Example 5).
FIG. 6 is a graph showing measurement results of an RNA copy number in culture cells in the case of adding the HCV particle formation promoter of the present invention to the cells (Example 6).
FIG. 7 shows results of confirmation of a protein-forming ability in culture cells by immunostaining in the case of adding the HCV particle formation promoter of the present invention to the cells (Example 6).

### MODE FOR CARRYING OUT INVENTION

The present invention relates to a HCV particle formation promoter, comprising as an active ingredient a statin or a pharmaceutically acceptable salt thereof.

Herein, the statin is a drug used as a therapeutic drug for hypercholesterolemia by inhibiting the function of 3-hydroxy-3-methylglutaryl coenzyme A reductase (HMG-CoA reductase), which is a rate-limiting enzyme of the mevalonate pathway, which is one of the cholesterol biosynthetic pathways, to suppress cholesterol biosynthesis in the liver, and the statin refers to lovastatin, simvastatin, fluvastatin sodium, pravastatin, atorvastatin calcium, pitavastatin calcium, rosuvastatin calcium, or the like.

Herein, the lovastatin has a CAS number of 75330-75-5 and refers to (1S,3R,7S,8S,8aR)-8-[2-((2R,4R)-4-hydroxy-6-oxotetrahydro-2H-pyran-2-yl)ethyl]-3,7-dimet hyl-1,2,3,7,8,8a-hexahydro-1-naphthyl, the simvastatin has a CAS number of 79902-63-9 and refers to

(1S,3R,7S,8S,8aR)-8-[2-[(2R,4R)-4-hydroxy-6-oxotetrahydropyran-2-yl]ethyl]-3,7-dimethyl-1,2,3,7,8,8a-hexahydro-1-naphthyl, the fluvastatin sodium has a CAS number of 93957-55-2 and refers to sodium (3R,5S,6E)-7-[3-(4-fluorophenyl)-1-(1-methylethyl)-1H-indol-2-yl]-3,5-dihydroxy-6-hepteno ate, the pravastatin has a CAS number of 81093-70-6 and refers to (1S,βR,δR,8aβ)-1,2,6,7,8,8a-hexahydro-β,δ,6β-trihydroxy-2β-methyl-8α-[(S)-2-methyl-1-oxo butoxy]-1β-naphthaleneheptanoate, the atorvastatin calcium has a CAS number of 134523-03-8 and refers to calcium (3R,5R)-7-[2-(4-fluorophenyl)-5-isopropyl-3-phenyl-4-[(phenylamino)carbonyl]-1H-pyrrol-1 -yl]-3,5-dihydroxyheptanoate, the pitavastatin calcium has a CAS number of 147511-69-1 and refers to (3R,5S,6E)-7-(2-cyclopropyl-4-(4-fluorophenyl)quinolin-3-yl)-3,5-dihydroxyhept-6-enoic acid, and the rosuvastatin calcium has a CAS number of 147098-20-2 and refers to calcium bis[(E)-7-[4-(4-fluorophenyl)-6-isopropyl-2-[methyl(methylsulfonyl)amino]pyrimidin-5-yl](3 R,5S)-3,5-dihydroxyhept-6-enoate].

As the statin of the present invention, one kind or a plurality of kinds may be selected from the above-mentioned statins and used. Preferred examples thereof include lovastatin, fluvastatin, simvastatin, atorvastatin, and pravastatin. More preferred examples thereof include lovastatin, fluvastatin, simvastatin, and atorvastatin. The active ingredient contained in the HCV particle formation promoter of the present invention may be the above-mentioned statin or a pharmaceutically acceptable salt thereof, or may be a hydrate thereof. The statin, the pharmaceutically acceptable salt thereof, and the hydrate thereof may be produced by synthesis, or may be produced with a microorganism.

The HCV particle formation promoter of the present invention may be a composition comprising as an active ingredient a statin, pharmaceutically acceptable salt thereof, and/or hydrate thereof produced by synthesis, or may be a culture extract of a microorganism capable of producing a statin, a pharmaceutically acceptable salt thereof, and/or a hydrate thereof. The culture extract of the microorganism may also be used without any further treatments as the HCV particle formation promoter of the present invention. In any of the cases, the active ingredient comprised in the HCV particle formation promoter of the present invention is a statin, a pharmaceutically acceptable salt thereof, and/or a hydrate thereof. When the culture extract of the microorganism capable of producing a statin, a pharmaceutically acceptable salt thereof, and/or a hydrate thereof is used without any further treatments as the HCV particle formation promoter of the present invention, the production of the HCV particle formation promoter can be easily performed, and a reduction in cost can be achieved, as compared to the case of synthesizing and purifying the statin, pharmaceutically acceptable salt thereof, and/or hydrate thereof.

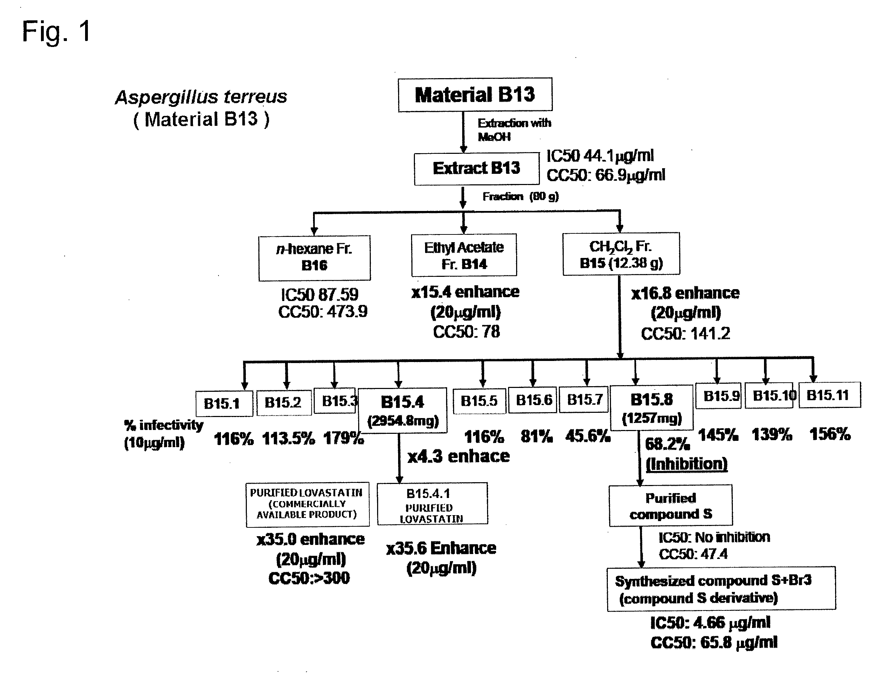

The statin, pharmaceutically acceptable salt thereof, and hydrate thereof as the active ingredient may be produced by synthesis, or may be acquired from a culture extract of a microorganism capable of biosynthesizing such ingredient. Methods of synthesizing the statin, pharmaceutically acceptable salt thereof, and hydrate thereof are not particularly limited, and methods known per se or all synthetic methods to be developed in the future are applicable thereto. The microorganism capable of producing a statin, a pharmaceutically acceptable salt thereof, and/or a hydrate thereof is not particularly limited, and for example, a microorganism capable of producing lovastatin is exemplified by a filamentous fungus belonging to the genus *Aspergillus*, particularly suitably *Aspergillus terreus* (see FIG. 1).

The pharmaceutically acceptable salt in the present invention is not particularly limited and may be exemplified by any salt known to a person skilled in the art, such as a sodium salt, a potassium salt, or a calcium salt. The HCV particle formation promoter of the present invention may comprise any other compound in addition to the above-mentioned statin, pharmaceutically acceptable salt thereof, and/or hydrate thereof as the active ingredient.

The dosage form of the HCV particle formation promoter of the present invention is not particularly limited as long as the statin, pharmaceutically acceptable salt thereof, and/or hydrate thereof as the active ingredient can be cultured together with HCV-infected cells. In consideration of the foregoing, the HCV particle formation promoter of the present invention may appropriately comprise, in addition to the active ingredient of the present invention, a pharmaceutically acceptable carrier, excipient, binder, lubricant, colorant, and the like known to a person skilled in the art. The HCV particle formation promoter of the present invention may be easily prepared by any formulation preparation method known to a person skilled in the art. Suitable examples of the carrier may include lactose, starch, sucrose, glucose, methylcellulose, magnesium stearate, mannitol, sorbitol, and croscarmellose sodium. Further, suitable examples of the binder include starch, gelatin, a natural saccharide such as glucose, anhydrous lactose, free flowing lactose, β-lactone, or a corn sweetener, natural or synthetic gum such as gum arabic, guar gum, tragacanth, or sodium alginate, carboxymethylcellulose, polyethylene glycol, and wax. In addition, examples of the lubricant to be used for these dosage forms include sodium oleate, sodium stearate, magnesium stearate, sodium benzoate, and sodium chloride.

The present invention also encompasses a method of enhancing the production of HCV particles, wherein the method comprises culturing HCV-infected cells together with the HCV particle formation promoter. Cells that may be used in the production of HCV particles of the present invention only need to be HCV-permissive cells. Herein, the HCV-permissive cells mean cells capable of replicating HCV genomic RNA and/or capable of being infected with HCV. The HCV-permissive cells are cells derived from liver cells or lymphoid cells, but are not limited thereto. Specific examples of the liver cells include, but not limited to, primary liver cells, Huh7 cells, RCYM1RC cells, 5-15RC cells, HepG2 cells, IMY-N9 cells, HeLa cells, and 293 cells. Specific examples of the lymphoid cells include, but not limited to, Molt4 cells, HPB-Ma cells, and Daudi cells. Preferred examples of the HCV-permissive cells include Huh7 cells, RCYM1RC cells, 5-15RC cells, HepG2 cells, and cell lines derived from these cells. Cells derived from Huh7 cells are particularly preferred and examples of such cells include Huh7.5 cells, Huh7.5.1 cells, and Huh7-it cells. In particular, it is suitable that the cells can be subjected to subculture. Further, eukaryotic cells are preferred and human cells are more preferred. As those cells, commercially available ones may be utilized, or ones available from cell repositories may be used. Cell lines derived from any cells (e.g., cancer cells or stem cells) may also be used. As cell lines derived from Huh7 cells, there are given, for example, Huh7.5 cells (Blight KJ et al., J. Virol., 76:13001-13014, 2002), Huh7.5.1 cells (Zhong J et al., Proc. Natl. Acad. Sci. USA, 102:9294-9299, 2005), and Huh7-it cells (Yu L et al., J. Virol. Methods, 169:380-384, 2010).

The addition of the HCV particle formation promoter of the present invention to HCV-infected HCV-permissive cells selected from the above-mentioned cells can promote the formation of HCV particles and enhance the production of the HCV particles. Specifically, HCV is inoculated into any cultured HCV-permissive cells selected from the above-mentioned cells so as to adsorb the HCV onto the cells. Then, the cells are cultured for from 24 to 72 hours, preferably from 36 to 72 hours, more preferably about 48 hours in a culture medium supplemented with the HCV particle formation promoter at a concentration of from 10 to 200 µg/ml, preferably from 20 to 50 µg/ml. Thus, the formation of HCV particles can be promoted, and the production of the HCV particles can be enhanced.

The capacity of HCV-infected cells to produce HCV particles may be confirmed by any known virus detection method. For example, a culture supernatant of HCV-permissive cells may be fractionated by a sucrose density gradient to detect virus particles. HCV-infected cells having replicated HCV genomic RNA express a HCV protein. Therefore, when the HCV protein can be detected by culturing HCV-infected cells, the cells can be estimated to have replicated HCV genomic RNA. Further, the HCV protein may be detected in accordance with any known protein detection method. Specifically, the HCV protein may be detected by the method of Kaito M et al., J. Gen. Virol., 75:1755-1760, 1994. A capacity to produce virus particles may be confirmed by confirming the number of infectious virus particles in a culture supernatant. Specifically, the number of infectious virus particles in a culture supernatant may be confirmed by: inoculating a culture supernatant comprising infectious virus particles into non-infected cells; fixing the cells from 18 to 48 hours, preferably about 24 hours after the inoculation; performing immunostaining by a specific antibody against a HCV protein; and measuring the number of staining-positive cells. More specifically, the confirmation may be performed by subjecting a culture supernatant comprising infectious virus particles to a reaction with an anti-HCV core protein antibody by an enzyme-linked immunosorbent assay (ELISA) method, followed by detection.

The analysis of HCV RNA to be replicated in the HCV-infected cells may be performed by a general molecular biological method. A method of extracting RNA from the cells may be a method known per se. Specifically, the amount or sequence of replicated RNA may be analyzed by a northern blot method, a ribonuclease protection assay method, a RT-PCR method, or the like. The northern blot method or a quantitative RT-PCR may be used in the case of quantifying RNA, and a sequence analysis method may be used in the case of analyzing an RNA sequence.

The HCV particles to be produced by the method of the present invention have an ability to infect HCV-permissive cells. Herein, the HCV-permissive cells mean cells capable of replicating HCV genomic RNA and/or capable of being infected with HCV. The HCV-permissive cells are cells derived from liver cells or lymphoid cells, but are not limited thereto. Specific examples of the liver cells include, but not limited to, primary liver cells, Huh7 cells, RCYM1RC cells, 5-15RC cells, HepG2 cells, IMY-N9 cells, HeLa cells, and 293 cells. Specific examples of the lymphoid cells include, but not limited to, Molt4 cells, HPB-Ma cells, and Daudi cells. Preferred examples of the HCV-permissive cells include Huh7 cells, RCYM1RC cells, 5-15RC cells, HepG2 cells, and cell lines derived from these cells. Cells derived from Huh7 cells are particularly preferred and examples of such cells include Huh7.5 cells, Huh7.5.1 cells, and Huh7-it cells.

A method of purifying HCV particles from a virus liquid comprising HCV particles obtained in the foregoing, such as a culture supernatant comprising infectious virus particles, is not particularly limited, and a method known per se or a method to be developed in the future is applicable thereto. For example, the purification may be performed by removing cells and cell debris by centrifugation and/or with a filter or the like, followed by one or a combination in any order of ultrafiltration concentration, chromatography, and density gradient centrifugation.

The present invention also encompasses a method of producing a HCV vaccine comprising as antigens the HCV particles to be produced by the method of the present invention. The present invention also encompasses a HCV vaccine produced by the method.

In the production of the HCV vaccine of the present invention, it is preferred to use HCV particles having inactivated infectivity. A method of inactivating the infectivity is not particularly limited as long as the method may be clinically used, and a method known per se or a method to be developed in the future may be adopted. For example, the inactivated infectivity may be achieved by adding and mixing an inactivating agent such as formalin, β-propiolactone, or glutardialdehyde into, for example, a suspension of the HCV particles produced by the present invention so that the inactivating agent reacts with the HCV particles (Appaiahgari MB et al., Vaccine, 22:3669-3675, 2004). Alternatively, the infectivity may be quickly inactivated by irradiating the HCV particles with UV light to lose the infectivity. According to the irradiation with UV light, the inactivation can be performed while reducing the influence on the protein and the like constituting the HCV particles. A radiation source of UV light to be used for performing the inactivation may be a general commercially available germicidal lamp, in particular, a 15-W germicidal lamp, but is not limited thereto.

An adjuvant is not particularly limited as long as the adjuvant may be used as an adjuvant for a vaccine and may be clinically used. And, an adjuvant known per se or an adjuvant to be developed in the future is applicable thereto. For example, aluminum hydroxide (Alum) or the like, which has already been approved for its use as an adjuvant for a vaccine, is desired, but there may be used any adjuvant that may be clinically used. Examples thereof include a CpG oligonucleotide and double-stranded RNA. Examples of the double-stranded RNA may include polyI:C, polyICLC, and polyIpolyC12U.

The HCV particle formation promoter of the present invention may also be utilized in a method of evaluating an anti-HCV agent candidate substance. Herein, an example of the anti-HCV agent is a substance having a suppressing effect on the production of HCV particles in cells or a suppressing effect on the release of HCV particles from cells. The anti-HCV agent candidate substance is not particularly limited as long as the substance is expected to exhibit such effects, and examples thereof include a protein, a peptide, and a low-molecular-weight compound.

The method of evaluating an anti-HCV agent candidate substance is achieved by: culturing HCV-infected cells together with the HCV particle formation promoter of the present invention and an anti-HCV agent candidate substance, or with an anti-HCV agent candidate substance alone, for from 36 to 72 hours; and comparing abilities to form HCV particles in the cultured HCV-infected cells. Specifically, the method may include the following steps 1) to 3):
1) adding the HCV particle formation promoter of the present invention and an anti-HCV agent candidate substance to HCV-infected cells;
2) culturing the hepatitis C virus-infected cells; and
3) evaluating an ability to inhibit formation of hepatitis C virus particles by measuring the amount of the hepatitis C virus particles after the culturing.

Examples of the cells that may be used in this case include the HCV-permissive cells described above.

### EXAMPLES

The contents of the present invention are more specifically described by way of Examples for further understanding of the present invention. However, it is apparent that the present invention is by no means limited to Examples shown below.

### (Example 1) HCV Particle Formation Promoter

In this example, a solution using an *Aspergillus terreus* culture extract (B13) as a raw material was used as the HCV particle formation promoter of the present invention. The HCV particle formation promoter according to this example was produced by a method illustrated in FIG. 1. In Examples to be described later, a crude lovastatin solution "B15" or purified lovastatin solution "B15.4.1" shown in FIG. 1 was used as the HCV particle formation promoter. The composition of B15.4.1 was investigated by a nuclear magnetic resonance (NMR) method and liquid chromatography-mass spectrometry (LC/MS). As a result, it was confirmed that lovastatin was obtained as a substantially pure product. A lovastatin amount was measured by weighing a purified authentic sample (B15.4.1) after drying with a precision analytical balance.

### (Example 2) HCV Infectivity Titer in the Case of Treating Culture Cells

In this example, the influence on the HCV infectivity titer in the case of adding the HCV particle formation promoter to Huh7-it cells was confirmed.

### 1) HCV Particle Formation Promoter

In this example, the purified lovastatin solution "B15.4.1" (FIG. 1) of Example 1 was used as the "HCV particle formation promoter." A lovastatin solution prepared by adding a culture medium to the HCV particle formation promoter to adjust each lovastatin concentration at the time of virus inoculation and at the time of culture to from 1.25 to 20 µg/ml was used in the following.

### 2) Culture Cells

In this example, Huh7-it cells, a highly HCV-permissive cell line derived from Huh7 cells, were used as culture cells. The medium used was Dulbecco's modified Eagle's medium supplemented with 10% fetal calf serum, non-essential amino acids, penicillin, and streptomycin, and the cells were cultured using a 48-well culture plate.

### 3) HCV Stock Solution

A HCV stock solution to be used for promoting the formation of HCV particles of the present invention was prepared by the following method. The HCV strain used was JFH1 strain (genome sequence: GenBank accession number AB047639). RNA having the same sequence as that of the full genome of the HCV JFH1 strain was introduced (transfected) into Huh7-it cells by an electroporation method (van den Hoff MJ et al., Nucleic Acids Res., 20:2902, 1992), and the culture supernatant 72 hours after the introduction was collected. The collected culture supernatant was passed through a 0.45-µm filter (Millipore) to remove contaminants, and then added to other Huh7-it cells. An infectivity titer was calculated by measuring the number of HCV-infected cells after 72 hours by a focus method, and a solution adjusted so as to have an infectivity titer of 5.4×10⁴ infectious units/ml was used as the HCV stock solution. The infectivity titer for preparing the HCV stock solution was calculated by an immunostaining method. An anti-HCV-core (clone CP 14) monoclonal antibody was used as a primary antibody, and a HRP-labeled goat anti-mouse antibody was used as a labeling antibody. Konica Immunostain HRP-1000 (Konica Minolta) was added, and the number of virus antigen-positive cell populations stained in blue (immunofocus; also called focus) was measured under a microscope. Thus, the infectivity titer was calculated.

### 4) HCV Infection Method

A mixture of the HCV stock solution (5.4×10⁴ infectious units/ml) prepared in the above-mentioned section 3) and the HCV particle formation promoter was inoculated into culture cells, and the virus was adsorbed to the cells at 37°C for 2 hours. Then, the virus liquid was removed, and the cells were washed three times with a culture medium. After that, in the same manner as described above, the cells were cultured for 48 hours with the addition of the HCV particle formation promoter (lovastatin concentration: 1.25 to 20 µg/ml). The virus was inoculated into the culture cells so that the multiplicity of infection (moi) was 0.1.

### 5) Virus Infectivity Titer Measurement Method

In order to confirm a virus-producing ability, the culturemedium of the infected cells was centrifuged at 10,000 rpm for 3 minutes, and the centrifugation supernatant was used as a sample liquid. The sample liquid was inoculated into non-HCV-infected Huh7-it cells. 24 hours after the inoculation, the cells were fixed and subjected to immunostaining using, as a primary antibody, patient serum that had been found to strongly react with the HCV protein in advance, and using, as a secondary antibody, an Alexa 488-labeled goat anti-human IgG antibody (Molecular Probe). The number of staining-positive cells was measured, and the HCV infectivity titer was measured.

### 6) Virus Infectivity Titer Measurement Results

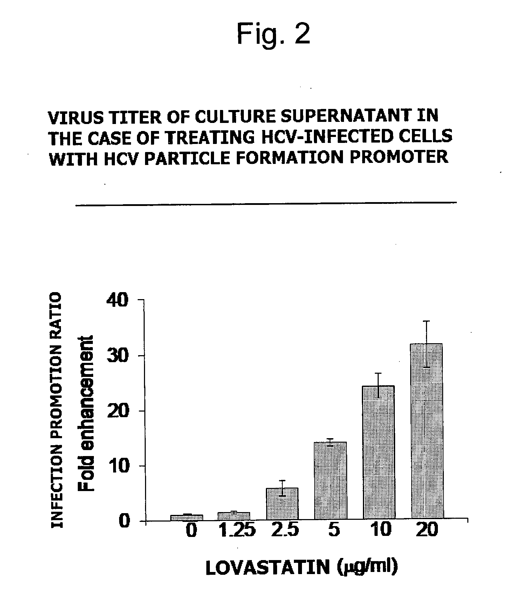

The results of the foregoing revealed that in the case of the culture in the presence of the purified lovastatin solution "B15.4.1" adjusted so as to contain 5 to 20 µg/ml of lovastatin, a high HCV infectivity titer was found in a lovastatin concentration-dependent manner for each sample liquid (FIG. 2).

### (Example 3) Study on Timing of Addition of HCV Particle Formation Promoter to Culture Cells (1)

In this example, the influence on the HCV infectivity titer in the case of adding the HCV particle formation promoter to Huh7-it cells during HCV adsorption for 2 hours and/or 48 hours after HCV infection was confirmed.

### 1) HCV Particle Formation Promoter

In this example, the crude lovastatin solution "B15" (FIG. 1) of Example 1 was used as the "HCV particle formation promoter." A lovastatin solution prepared by adding a culture medium to the HCV particle formation promoter to adjust each lovastatin concentration at the time of virus inoculation and at the time of culture to from 1 to 50 µg/ml was used in the following.

### 2) Culture Cells

In this example, in the same manner as in Example 2, Huh7-it cells were cultured and used.

### 3) Timing of Addition of HCV Particle Formation Promoter

In this example, HCV was inoculated into culture cells so as to achieve moi=0.1 as in Example 2, and the HCV particle formation promoter was added at the following timings i) to iii).

### i) During Adsorption + after Adsorption

A mixture of HCV and the HCV particle formation promoter was inoculated into culture cells, and the virus was adsorbed to the cells at 37°C for 2 hours. Then, the virus liquid was removed, and the cells were washed three times with a culture medium. After that, the cells were cultured for 46 hours with the addition of the HCV particle formation promoter containing lovastatin.

### ii) After Adsorption Only

HCV was inoculated into culture cells and adsorbed to the cells at 37°C for 2 hours. Then, the virus liquid was removed, and the cells were washed three times with a culture medium. After that, the cells were cultured for 46 hours with the addition of a culture medium supplemented with the HCV particle formation promoter.

### iii)) During Adsorption Only

A mixture of HCV and the HCV particle formation promoter was inoculated into culture cells, and the virus was adsorbed to the cells at 37°C for 2 hours. Then, the virus liquid was removed, and the cells were washed three times with a culture medium. After that, the cells were cultured for 46 hours with the addition of a culture medium free of the HCV particle formation promoter.

### 4) Virus Infectivity Titer Measurement Method

A sample for confirming a virus-producing ability was prepared in the same manner as in Example 2, and was measured for its virus infectivity titer in the same manner as in Example 2.

### 5) Virus Infectivity Titer Measurement Results

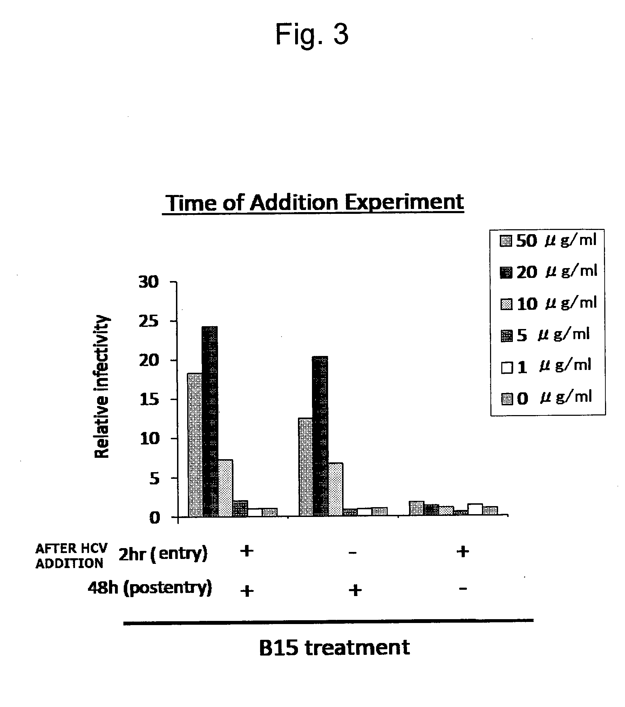

From the results of the foregoing, irrespective of the presence or absence of the HCV particle formation promoter during the HCV adsorption, when the cells were cultured with the addition of the HCV particle formation promoter after the HCV adsorption, a high virus infectivity titer was found in a concentration-dependent manner in the presence of 5 to 20 µg/ml of lovastatin for each sample liquid (FIG. 3).

### (Example 4) Study on Timing of Addition of HCV Particle Formation Promoter to Culture Cells (2)

In this example, the influence on the HCV infectivity titer in the case of adding lovastatin as the HCV particle formation promoter to Huh7-it cells during HCV adsorption for 2 hours or 48 hours after HCV infection was confirmed.

### 1) HCV Particle Formation Promoter

In this example, the purified lovastatin solution "B15.4.1" (FIG. 1) of Example 1 was used as the "HCV particle formation promoter." A lovastatin solution prepared by adding a culture medium to the HCV particle formation promoter to adjust each lovastatin concentration at the time of virus inoculation and at the time of culture to 20 µg/ml was used in the following.

### 2) Culture Cells

In this example, in the same manner as in Example 2, Huh7-it cells were cultured and used.

### 3) Timing of Addition of HCV Particle Formation Promoter

In this example, HCV was inoculated into culture cells so as to achieve moi=0.1 as in Example 2, and the HCV particle formation promoter was added at the following timings i) to iii).

### i) During Adsorption + after Adsorption

A mixture of HCV and the HCV particle formation promoter was inoculated into culture cells, and the virus was adsorbed to the cells at 37°C for 2 hours. Then, the virus liquid was removed, and the cells were washed three times with a culture medium. After that, the cells were cultured for 46 hours with the addition of a culture medium supplemented with the HCV particle formation promoter.

### ii) During Adsorption Only

A mixture of HCV and the HCV particle formation promoter was inoculated into culture cells, and the virus was adsorbed to the cells at 37°C for 2 hours. Then, the virus liquid was removed, and the cells were washed three times with a culture medium. After that, the cells were cultured for 46 hours with the addition of a medium free of the HCV particle formation promoter.

### iii) After Adsorption Only

HCV was inoculated into culture cells and adsorbed to the cells at 37°C for 2 hours. Then, the virus liquid was removed, and the cells were washed three times with a culture medium. After that, the cells were cultured for 46 hours with the addition of a culture medium supplemented with the HCV particle formation promoter.

### 4) Virus Infectivity Titer Measurement Method

A sample for confirming a virus-producing ability was prepared in the same manner as in Example 2, and was measured for its virus infectivity titer in the same manner as in Example 2.

### 5) Virus Infectivity Titer Measurement Results

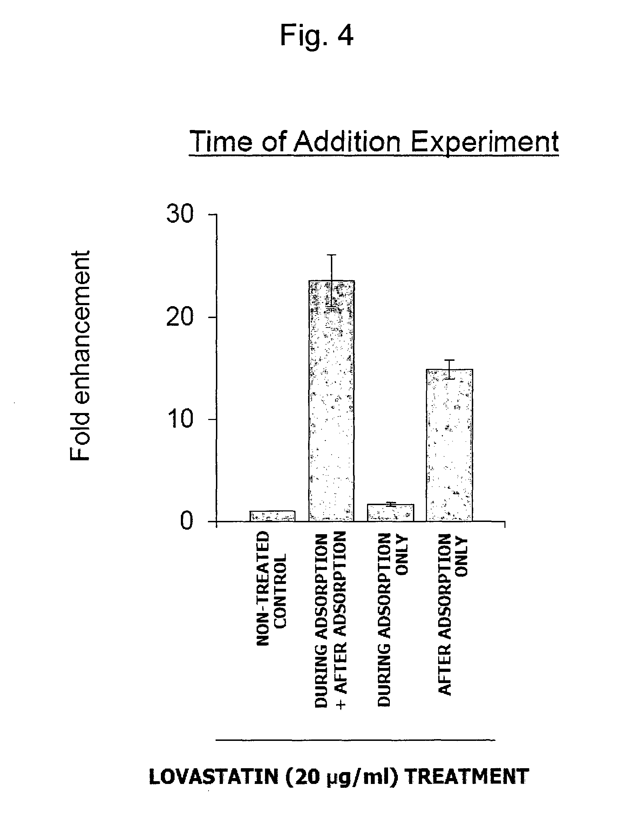

From the results of the foregoing, irrespective of the presence or absence of the HCV particle formation promoter during the HCV adsorption, when the cells were cultured with the addition of the HCV particle formation promoter after the HCV adsorption, a high virus infectivity titer was found (FIG. 4).

### (Example 5) Confirmation of HCV Particle Formation inside and outside Culture Cells

In this example, culture cells were prepared in the same manner as in Example 2 to confirm the influence on the HCV infectivity titer inside and outside Huh7-it cells in the case of adding the HCV particle formation promoter to the cells over a period of from during HCV adsorption to 48 hours after HCV infection. In this example, the purified lovastatin solution "B 15.4.1" (FIG. 1) of Example 1 was used as the HCV particle formation promoter. A lovastatin solution prepared by adding a culture medium to the HCV particle formation promoter to adjust each lovastatin concentration at the time of virus inoculation and at the time of culture to 20 µg/ml was used in the following. A system containing dimethylsulfoxide (DMSO) in place of the HCV particle formation promoter was used as a control.

A sample for measuring an extracellular HCV infectivity titer was prepared in the same manner as in Example 2. A sample for measuring an intracellular HCV infectivity titer was prepared by a freeze-thawing method. First, infected cells were washed with PBS and then detached by trypsin/EDTA treatment, followed by centrifugation to collect a cell pellet. The cell pellet was washed once with PBS, suspended in 0.5 ml of a culture medium, and freeze-thawed at -80°C three times repeatedly, followed by centrifugation at 12,000 rpm for 5 minutes. The resultant supernatant was used as a sample for measurement. Each sample was measured for its virus titer in the same manner as in Example 2.

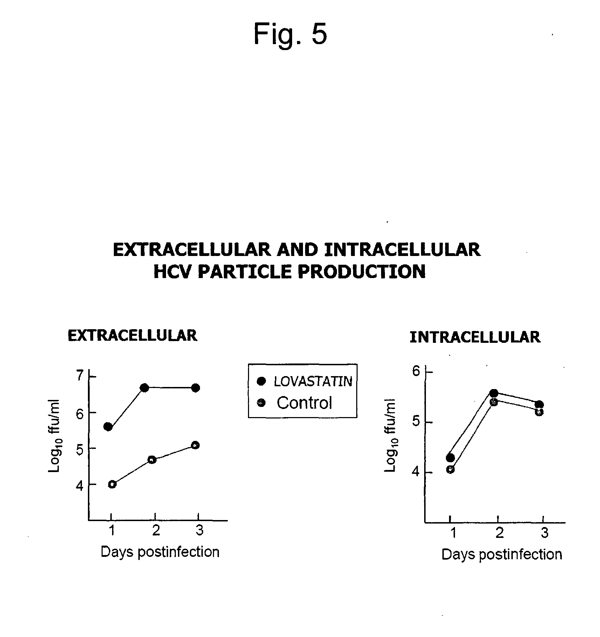

From the results of the foregoing, substantially no difference in intracellular HCV particle-producing ability was found between the system treated with the HCV particle formation promoter and the control. On the other hand, the system treated with the HCV particle formation promoter was found to have a significantly high extracellular HCV particle-producing ability, which was from 20 times to 30 times as high as that of the control (FIG. 5).

### (Example 6) Confirmation of HCV RNA Copy Number in Culture Cells

In this example, culture cells were prepared in the same manner as in Example 2 to confirm the influence on the HCV RNA copy number in the culture cells on days 1,2, 3, and 4 after HCV infection in the case of adding the HCV particle formation promoter to Huh7-it cells over a period of from during HCV adsorption to 48 hours after HCV infection. In this example, the purified lovastatin solution "B15.4.1" (FIG. 1) of Example 1 was used as the HCV particle formation promoter. A lovastatin solution prepared by adding a culture medium to the HCV particle formation promoter to adjust each lovastatin concentration at the time of virus inoculation and at the time of culture to 20 µg/ml was used in the following. A system containing DMSO in place of the HCV particle formation promoter was used as a control.

A sample for measuring an intracellular HCV RNA copy was prepared in the same manner as in Example 5. Total RNA was extracted from infected cells using Trizol^{®} /Trizol^{®}-LS (Invitrogen). cDNA was synthesized from the total RNA by performing a reverse transcription reaction at 37°C for 15 minutes and an enzyme inactivation reaction at 98°C for 5 minutes through the use of a ReverTra Ace^{®} qPCR-Kit (Toyobo). The HCV RNA copy number was measured by a quantitative RT-PCR method using a LightCycler^{®} 480 real-time PCR system. Primers for the NS3 site, having nucleotide sequences shown in SEQ ID NOS: 1 and 2, were used as HCV-specific primers, and a solution containing 10 µl of 2×SYBR Premix ExTaq (Takara), 0.4 µl of each primer (10 pmol/µl), 2 µl of the cDNA template, and 7.2 µl of sterile distilled water was used as a PCR reaction solution. The conditions of PCR were as follows: heat denaturation at 95°C for 10 seconds was performed and then a cycle composed of 95°C for 10 seconds and 60°C for 20 seconds was repeated 40 times.
(SEQ ID NO: 1) 5'-CTTTGACTCCGTGATCGACT-3'
(SEQ ID NO: 2) 5'-CCCTGTCTTCCTCTACCTG-3'

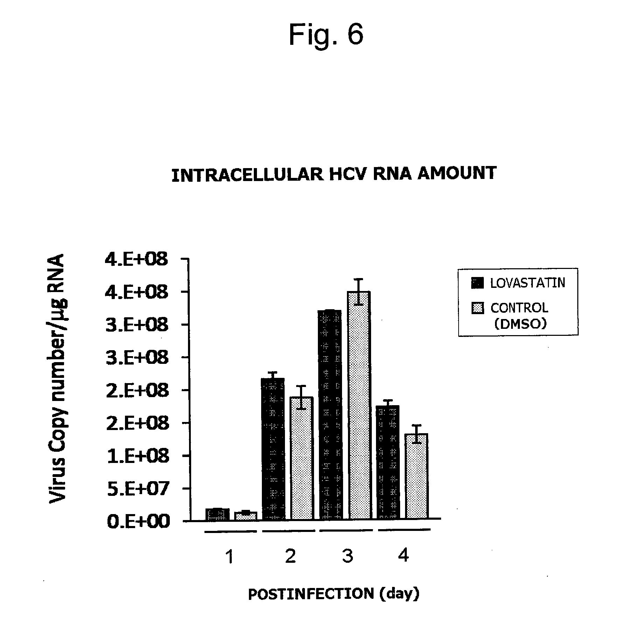

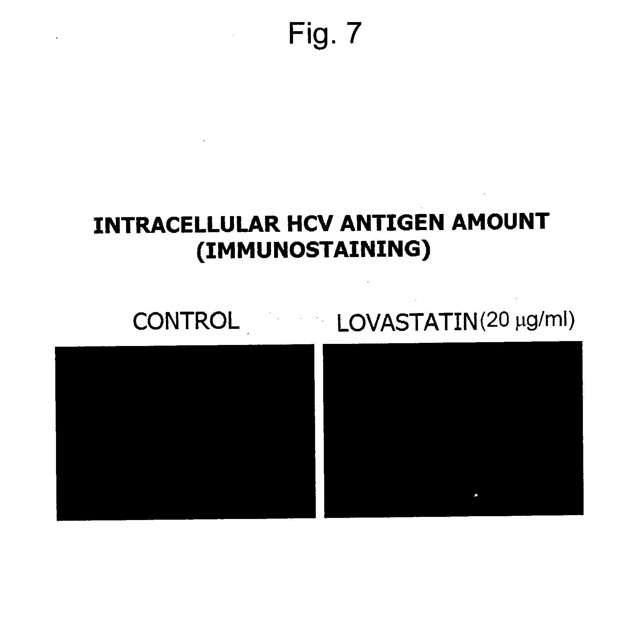

The experimental results of the foregoing revealed that an intracellular HCV RNA replication amount was not affected by the presence or absence of the HCV particle formation promoter (FIG. 6). In addition, the immunostaining revealed that an intracellular HCV protein (antigen) synthesis amount was also not affected by the presence or absence of lovastatin (FIG. 7). It was estimated from those experimental results that the HCV production-enhancing effect of lovastatin was not exhibited at the stages of HCV RNA replication and HCV protein synthesis, but was exhibited at the subsequent stages, i.e., the stages of HCV particle formation and release.

### (Example 7) HCV Particle Production-promoting Effects of Various Statins

In this example, commercially available various statins were observed for their HCV particle production-promoting effects.

### 1) HCV Particle Formation Promoter

In this example, a commercially available statin formulation was used as the HCV particle formation promoter. The following statins were used: lovastatin (mevinolin: manufactured by Sigma-Aldrich; CAS number 75330-75-5), fluvastatin sodium (manufactured by Wako Pure Chemical Industries, Ltd.; CAS number 93957-55-2), simvastatin (manufactured by Sigma-Aldrich.; CAS number 79902-63-9), atorvastatin calcium trihydrate (manufactured by Wako Pure Chemical Industries, Ltd.; CAS number 134523-03-8), and pravastatin sodium salt hydrate (manufactured by Wako Pure Chemical Industries, Ltd.; CAS number 81131-70-6). Each of the statins was diluted with a culture medium to adjust its concentration at the time of culture to a concentration shown in Table 1. DMSO (1 µg/ml) in place of the HCV particle formation promoter was used as a control.

### 2) Culture Cells

In this example, in the same manner as in Example 2, Huh7-it cells were cultured and used.

### 3) Timing of Addition of HCV Particle Formation Promoter

In this example, HCV was inoculated into culture cells so as to achieve moi=0.1, and the virus was adsorbed to the cells at 37°C for 2 hours. Then, the virus liquid was removed, and the cells were washed three times with a culture medium. After that, the cells were cultured for 46 hours with the addition of a culture medium supplemented with the HCV particle formation promoter.

### 4) Virus Infectivity Titer Measurement Method

A sample for confirming a virus-producing ability was prepared in the same manner as in Example 2, and was measured for its virus infectivity titer in the same manner as in Example 2.

### 5) Virus Infectivity Titer Measurement Results

Table 1 shows the results of the foregoing. As a result, each of the statins was found to have a HCV particle formation promoter effect.

**[Table 1]**

| HCV particle production-promoting effects of various statins (commercially available products) | | |
|---|---|---|
| Statin | Concentration (µg/ml) | Promotion ratio |
| Control (DMSO) | 1.0 | 1.0 |
| | | |
| Lovastatin | 1.25 | 0.8 |
| | 2.5 | 3.5 |
| | 5.0 | 6.1 |
| | 20.0 | 11.1 |
| | | |
| Fluvastatin | 1.25 | 9.2 |
| | 2.5 | 14.3 |
| | 5.0 | 15.2 |
| | 20.0 | 7.9 |
| | | |
| Simvastatin | 1.25 | 8.5 |
| | 2.5 | 14.8 |
| | 5.0 | 19.5 |
| | 20.0 | 4.1 |
| | | |
| Atorvastatin | 1.25 | 0.9 |
| | 2.5 | 4.3 |
| | 5.0 | 9.4 |
| | 20.0 | 9.7 |
| | | |
| Pravastatin | 5.0 | 0.6 |
| | 20.0 | 2.7 |
| | 80.0 | 17.2 |
| | 200.0 | 24.7 |

### INDUSTRIAL APPLICABILITY

As described in detail above, the production efficiency of HCV particles in a cell culture medium can be expected to be improved by 10 times or more by culturing HCV-infected cells with the addition of the HCV particle formation promoter of the present invention. This enables the HCV vaccine to be produced effectively and efficiently. Further, the anti-HCV agent can be evaluated by: culturing HCV-infected cells together with the HCV particle formation promoter of the present invention and the anti-HCV agent candidate substance; and measuring the virus titer and/or HCV RNA copy number in a cell culture medium. Still further, the HCV vaccine can be efficiently produced using the HCV particles obtained by the present invention.

## Claims

1. A hepatitis C virus particle formation promoter, comprising as an active ingredient a statin or a pharmaceutically acceptable salt thereof.

2. The hepatitis C virus particle formation promoter according to claim 1, wherein the statin comprises one or more statins selected from the group consisting of lovastatin, fluvastatin, simvastatin, atorvastatin, and pravastatin.

3. The hepatitis C virus particle formation promoter according to claim 1 or 2, wherein the hepatitis C virus particle formation promoter comprises a culture extract of a microorganism capable of producing a statin or a pharmaceutically acceptable salt thereof.

4. The hepatitis C virus particle formation promoter according to claim 3, wherein the microorganism is a filamentous fungus belonging to a genus *Aspergillus.*

5. A method of enhancing production of hepatitis C virus particles, wherein the method comprises culturing a hepatitis C virus-infected cell in a presence of the hepatitis C virus particle formation promoter according to any one of claims 1 to 4.

6. The method of enhancing production of hepatitis C virus particles according to claim 5, wherein the culturing is performed with addition of the hepatitis C virus particle formation promoter according to any one of claims 1 to 4 after formation of a hepatitis C virus protein in the hepatitis C virus-infected cell.

7. A method of producing a hepatitis C virus vaccine, wherein the method comprises inactivating a hepatitis C virus produced by the method of enhancing production of hepatitis C virus particles according to claim 5 or 6.

8. A hepatitis C virus vaccine, which is produced by the method of producing a hepatitis C virus vaccine according to claim 7.

9. A method of evaluating an anti-hepatitis C virus agent candidate substance,
wherein the method comprises:
culturing a hepatitis C virus-infected cell together with an anti-hepatitis C virus agent candidate substance in a presence of the hepatitis C virus particle formation promoter according to any one of claims 1 to 4; and
evaluating the anti-hepatitis C virus agent candidate substance for an ability to inhibit formation of hepatitis C virus particles.

10. The method of evaluating an anti-hepatitis C virus agent candidate substance according to claim 9, wherein the method comprises the steps of:
1) adding the hepatitis C virus particle formation promoter according to any one of claims 1 to 4 and an anti-hepatitis C virus agent candidate substance to a hepatitis C virus-infected cell;
2) culturing the hepatitis C virus-infected cell; and
3) evaluating the anti-hepatitis C virus agent candidate substance for an ability to inhibit formation of a hepatitis C virus particle by measuring an amount of the hepatitis C virus particles after the culturing.


SEQUENCE LISTING

<110>  NATIONAL UNIVERSITY CORPORATION KOBE UNIVERSITY
       TORAY INDUSTRIES, INC.
       TOKYO METROPOLITAN ORGANIZATION FOR MEDICAL RESEARCH
       University of Indonesia
       INDONESIAN INSTITUTE OF SCIENCES(LIPI)

<120>  A promoting agent for forming particles of hepatitis C virus and a
method for producing particles of hepatitis C virus

<130>  GP13-1009PCT

<150>  JP P2012-215474
<151>  2012-09-28

<160>  2

<170>  PatentIn version 3.1

<210>  1
<211>  20
<212>  DNA
<213>  Artificial

<220>
<223>  Synthesizes primer DNA for amplify hepatitis C virus genome

<400>  1
ctttgactcc gtgatcgact                                                20


<210>  2
<211>  19
<212>  DNA
<213>  Artificial

<220>
<223>  Synthesizes primer DNA for amplify hepatitis C virus genome

<400>  2
ccctgtcttc ctctacctg                                                 19